(19) **Europäisches Patentamt / European Patent Office / Office européen des brevets**

(11) EP 2 736 355 B1

(12) **FASCICULE DE BREVET EUROPEEN**

(45) Date de publication et mention de la délivrance du brevet:
**01.11.2017 Bulletin 2017/44**

(21) Numéro de dépôt: **12740977.9**

(22) Date de dépôt: **30.07.2012**

(51) Int Cl.:
| | |
|---|---|
| *A61K 8/92* (2006.01) | *A61Q 17/04* (2006.01) |
| *A61Q 19/08* (2006.01) | *C11B 1/10* (2006.01) |
| *C11B 7/00* (2006.01) | *A61K 36/00* (2006.01) |
| *A23L 33/105* (2016.01) | *A23L 33/11* (2016.01) |
| *A23L 33/15* (2016.01) | *A61K 8/99* (2017.01) |
| *A61Q 19/00* (2006.01) | *A61Q 19/06* (2006.01) |
| *A61K 8/97* (2017.01) | *C11B 1/04* (2006.01) |
| *A23D 9/00* (2006.01) | *C11B 9/02* (2006.01) |
| *A61K 36/48* (2006.01) | *A61K 36/54* (2006.01) |

(86) Numéro de dépôt international:
**PCT/EP2012/064901**

(87) Numéro de publication internationale:
**WO 2013/014298 (31.01.2013 Gazette 2013/05)**

(54) **EXTRACTION SOLIDE / LIQUIDE AVEC UN SOLVANT COMPRENANT ENTRE 5 ET 8 ATOMES DE CARBONE ET 1 OU 2 ATOMES D'OXYGENE**

FEST-/FLÜSSIGEXTRAKTION MIT EINEM LÖSUNGSMITTEL MIT ZWISCHEN 5 UND 8 KOHLENSTOFFATOMEN UND 1 ODER 2 SAUERSTOFFATOMEN

SOLID/LIQUID EXTRACTION WITH A SOLVENT COMPRISING BETWEEN 5 AND 8 CARBON ATOMS AND 1 OR 2 OXYGEN ATOMS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.07.2011 FR 1156935**

(43) Date de publication de la demande:
**04.06.2014 Bulletin 2014/23**

(73) Titulaire: **Laboratoires Expanscience**
**92048 Paris La Défense Cedex (FR)**

(72) Inventeurs:
- **MERCIER, Eglantine**
  **F-78120 Rambouillet (FR)**
- **LEGRAND, Jacques**
  **F-61290 Neuilly Sur Eure (FR)**
- **SAUNOIS, Alex**
  **F-28210 Nogent-le-Roi (FR)**

(74) Mandataire: **Regimbeau**
**20, rue de Chazelles**
**75847 Paris Cedex 17 (FR)**

(56) Documents cités:
| | |
|---|---|
| EP-A1- 1 733 731 | WO-A1-2011/122278 |
| US-A- 4 549 990 | US-A- 5 679 393 |

Remarques:
Le dossier contient des informations techniques présentées postérieurement au dépôt de la demande et ne figurant pas dans le présent fascicule.

**Description**

La présente invention concerne un procédé d'extraction solide / liquide d'un extrait naturel, notamment une huile ou un beurre, à partir d'une matière solide végétale ou d'un micro-organisme, ledit extrait, notamment huile ou beurre, comprenant en particulier une teneur importante en insaponifiable.

Les insaponifiables ou fractions insaponifiables d'un corps gras sont constitués de composés qui, après action prolongée d'une base alcaline, restent insolubles dans l'eau et peuvent être extraits par un solvant organique.

La plupart des insaponifiables d'huiles végétales comprennent des grandes familles de substances. Parmi ces grandes familles on peut citer les hydrocarbures saturés ou insaturés, les alcools aliphatiques ou terpéniques, les stérols, les tocophérols, les pigments caroténoïdes, xanthophiles, ainsi qu'une ou deux familles spécifiques dans le cas de certaines huiles.

Les procédés usuels d'obtention des insaponifiables des huiles végétales visent à extraire tout ou partie des grandes familles qui les composent, permettant de préparer les fractions partielles ou totales des insaponifiables.

Les fractions partielles ou totales d'insaponifiables sont notamment recherchées pour leurs propriétés pharmacologiques, cosmétiques et nutritionnelles.

Les procédés usuels d'obtention des insaponifiables comprennent entre autre une étape de saponification de la matière grasse et une extraction du produit cible (l'insaponifiable) par un solvant organique.

Les solvants les plus communément utilisés pour extraire les huiles ou les beurres, en particulier riches en insaponifiable, de matières solides végétales ou de micro-organismes sont les solvants aliphatiques, et en particulier l'hexane, ainsi que les esters d'acides gras, comprenant plus de 10 atomes de carbone, et en particulier le méthyl palmitate, le méthyl stéarate et l'éthyl décanoate.

Le brevet US 5,679,393 décrit un procédé de préparation d'une fraction d'huile enrichie en insaponifiable. Ce document décrit en particulier un procédé d'extraction solide/liquide d'une matière grasse d'origine végétale telle qu'un beurre de karité, à l'aide d'un solvant aliphatique ou aryle ester comprenant au moins 10 atomes de carbone.

La demande internationale WO 2011/122278 décrit un procédé d'extraction solide/liquide d'une matière solide végétale en utilisant de l'acétone ou la méthyléthylcétone en tant que solvant d'extraction.

L'hexane présente notamment l'inconvénient d'être toxique, il est notamment classé CMR de classe 3 dans la liste CMR UE1 ou dans la liste CMR UE2.

L'hexane présente encore l'inconvénient d'être dangereux en terme de manipulation, notamment du fait de ses propriétés physico-chimiques, en particulier de son point éclair (-23,3°C) et/ou de sa température d'auto inflammation (233,9°C).

Les esters d'acides gras présentent l'inconvénient d'avoir un point d'ébullition élevé (généralement supérieur à 185°C) ; leur élimination par distillation est coûteuse en énergie et, du fait des fortes températures requises, peut nuire à la qualité des fractions insaponifiables extraites.

Enfin, les procédés impliquant ces solvants aliphatiques classiques, et notamment l'hexane, peuvent être insatisfaisants en termes de rendement, par rapport à l'huile ou au beurre et/ou par rapport à la teneur de l'huile ou du beurre obtenue en insaponifiable, notamment en termes de taux d'extraction d'une ou de plusieurs fractions insaponifiables particulières, de sélectivité, de simplicité, de coût, de toxicité, d'écotoxicité, de commodité, de nombre d'étapes, notamment d'extraction, et/ou de rapidité.

Par ailleurs, tant d'un point de vue économique que d'un point de vue environnemental, les procédés d'obtention d'huiles peuvent requérir l'utilisation de quantités de solvants organiques non adaptées pour la viabilité du procédé, présenter un nombre d'étapes d'extraction insatisfaisants et/ou être trop lents.La présente invention vise donc à résoudre en tout ou partie les problèmes évoqués ci-dessus. En particulier, l'invention vise à fournir un procédé présentant un rendement global plus élevé, permettant l'obtention d'une huile ou d'un beurre avec une teneur en insaponifiable plus importante ou présentant un profil particulier, plus économique, plus direct, plus amical pour l'environnement, nécessitant une quantité de solvant organique plus faible, plus facile à mettre en oeuvre, plus rapide, générant des conditions moins toxiques, permettant l'obtention d'huiles ou de beurres, notamment présentant une teneur importante en insaponifiable, avec un rendement et/ou une sélectivité au moins comparables, voire supérieurs, aux procédés déjà existants.

En particulier, il est souhaitable que le(s) solvant(s) impliqué(s) soi(en)t moins toxique(s), notamment non-classé(s) CMR, notamment CMR UE2, et/ou permette(nt) d'extraire les huiles ou les beurres avec un rendement et/ou une sélectivité au moins comparables aux rendements et sélectivités obtenus en utilisant les solvants aliphatiques classiques, et notamment l'hexane, et les esters d'acides gras, notamment le méthyl stéarate.

Les solvants dits « classés CMR » peuvent être ceux qui sont présentés dans la liste en annexes de la directive 2009/2/CE du 15 janvier 2009, cette première liste étant appelée ci-après « liste CMR UE1 », ceux listés dans la Classification européenne réglementaire des produits chimiques cancérogènes, mutagènes et toxiques pour la reproduction - 31e ATP, 2009, cette deuxième liste étant appelée ci-après « liste CMR UE2 », et/ou ceux listés dans la liste « Chemicals known or suspected to cause cancer or reproductive toxicity » du 1er septembre 2009 établie par le « California department of public health, occupational health branch, California safe cosmetic program » liée à la « California Safe Cosmetics Act of 2005 », cette troisième liste étant appelée ci-après « liste CMR US ».

Lorsque dans le présent texte est utilisée l'expression liste CMR UE, on entend liste CMR UE1 et/ou CMR UE2, et en particulier CMR UE2.

Les solvants utilisés dans le cadre de la présente invention sont ainsi dépourvus des familles de solvants et solvants suivants :

- certains alcanes, tels que l'hexane, l'heptane,...
- certains hydrocarbures aromatiques, tels que le naphtalène,
- certains solvants halogénés, notamment les solvants chlorés (1,2-dichloroéthane ou DCE, trichloroéthane, dichlorométhane, trichlorométhane (chloroforme), dichloroéthylène, tétrachlorure de carbone,...), ou encore le 1-chlorobutane.

Le procédé selon l'invention peut en particulier viser à améliorer le rendement en extrait naturel, notamment en huile ou en beurre, et/ou la teneur en insaponifiable présent dans cet extrait.

La présente invention a ainsi pour objet un procédé d'extraction solide / liquide d'un extrait naturel, notamment comprenant ou consistant en une huile ou un beurre, en particulier présentant une teneur importante en insaponifiable, contenu dans au moins une matière solide végétale ou un micro-organisme comprenant au moins les étapes suivantes :

- extraction solide / liquide d'au moins une matière solide végétale ou d'un micro-organisme par un premier système de solvants comprenant une teneur en solvant choisi parmi les solvants comprenant entre 5 et 8 atomes de carbone et un ou deux atomes d'oxygène sous forme soit de fonction éther, soit de fonction cétone, soit de fonction ester, d'au moins 50% en volume par rapport au volume total du premier système de solvant, lesdits solvants étant choisis parmi les méthylcétones, notamment le méthyl isobutyl cétone (MIBK), le 2-heptanone, les propionates, notamment l'éthyl propionate, le n-butyl propionate, l'isoamyl propionate, les propyléthers, notamment le diisopropyl éther (DIPE), et leurs mélanges,
- récupération d'un extrait naturel, notamment comprenant ou consistant en une huile ou en un beurre, en particulier enrichi en insaponifiable.

Par « extrait naturel », on entend au sens de la présente invention un extrait végétal, minéral ou animal.

Par « système de solvants », on entend au sens de la présente invention un seul solvant ou un mélange de solvants.

Par « micro-organisme », on entend tout organisme vivant microscopique, tel que les bactéries et/ou des champignons, notamment des levures et des moisissures.

Typiquement, l'extraction solide / liquide est réalisée par la mise en contact d'au moins une matière solide végétale et/ou d'un micro-organisme avec le premier système de solvant à chaud.

Suivant un mode de réalisation particulier, l'extraction solide / liquide est réalisée au moyen d'un Soxhlet. Dans ce cas particulier, le solvant est avantageusement porté à reflux pour la conduite de l'extraction.

Lors de l'étape de récupération, les extraits naturels peuvent être récupérés notamment via des procédés de désolvantation, de filtration et/ou de cristallisation.

Lorsque le premier système de solvant comprend une teneur en un solvant ou un mélange de solvants choisis dans une liste de X %, cela signifie que le pourcentage complémentaire correspond à un ou plusieurs solvants organique(s) ne figurant pas dans cette liste.

Selon un mode de réalisation particulier, le premier système de solvant est dépourvu de tertbutyl éthers, en particulier de ETBE et /ou de MTBE, ou encore de terpènes, en particulier de limonène et d'alpha-pinène.

Ledit solvant comprenant entre 5 et 8 atomes de carbone et un ou deux atomes d'oxygène est choisi parmi les méthylcétones, notamment le méthyl isobutyl cétone ou MIBK, la 2-heptanone, les propionates, notamment l'éthyl propionate, le n-butyl propionate, l'isoamyl propionate, les propyléthers, notamment le diisopropyl éther ou DIPE, et leurs mélanges.

Les numéros CAS de ces différents solvants sont les suivants : méthyl isobutyl cétone ou MIBK: 108-10-1 ; 2-heptanone : 110-43-0 ; éthyl propionate: 105-37-3 ; butyl propionate : 590-01-2 ; isoamyl propionate : 105-68-0 ; diisopropyl éther ou DIPE : 108-20-3.

Par « teneur importante en insaponifiable », on entend selon la présente invention que l'huile ou le beurre comprend au moins 1 % en masse, notamment au moins 2 % en masse, et en particulier au moins 3 % en masse des composés insaponifiables présents initialement dans la matière solide.

Le procédé selon l'invention peut en particulier être dépourvu d'une étape de complexation impliquant le premier système de solvant.

Le premier système de solvants peut comprendre une teneur de solvant choisi parmi les solvants comprenant entre 5 et 8 atomes de carbone et un ou deux atomes d'oxygène sous forme soit de fonction éther, soit de fonction cétone, soit de fonction ester, en particulier choisi parmi les méthylcétones, notamment le MIBK, le 2-heptanone, les propionates, notamment l'éthyl propionate, le n-butyl propionate, l'isoamyl propionate, les propyléthers, notamment le DIPE, et leurs mélanges, d'au moins 60%, notamment au moins 75%, en particulier au moins 90%, plus particulièrement au moins 95%, encore plus particulièrement au moins 99%, en volume par rapport au volume total du premier système de solvant.

Le premier système de solvants est constitué de solvant comprenant entre 5 et 8 atomes de carbone et un ou deux atomes d'oxygène sous forme soit de fonction éther, soit de fonction cétone, soit de fonction ester, choisi parmi les méthylcétones, notamment le MIBK, le 2-heptanone, les propionates, notamment l'éthyl propionate, le n-butyl propionate, l'isoamyl propionate, les propyléthers, notamment le DIPE, ou d'un mélange de ceux-ci.

Le premier système de solvants peut comprendre une teneur en un solvant choisi parmi les solvants comprenant entre 5 et 8 atomes de carbone et un ou deux atomes d'oxygène sous forme soit de fonction éther, soit de fonction cétone, soit de fonction ester, en particulier choisi parmi les méthylcétones, notamment le MIBK, le 2-heptanone, les propionates, notamment l'éthyl propionate, le n-butyl propionate, l'isoamyl propionate, les propyléthers, notamment le DIPE, et leurs mélanges, d'au moins 60%, notamment au moins 75%, en particulier au moins 90%, plus particulièrement au moins 95%, encore plus particulièrement au moins 99%, en volume par rapport au volume total du premier système de solvants.

Selon une variante, le premier système de solvants est constitué d'un solvant choisi parmi les solvants comprenant entre 5 et 8 atomes de carbone et un ou deux atomes d'oxygène sous forme soit de fonction éther, soit de fonction cétone, soit de fonction ester, en particulier choisi parmi les méthylcétones, notamment le MIBK, le 2-heptanone, les propionates, notamment l'éthyl propionate, le n-butyl propionate, l'isoamyl propionate, et les propyléthers, notamment le DIPE.

Selon un mode de réalisation particulier, le premier système de solvant présente une densité inférieure à 1, et notamment inférieure ou égale à 0,9.

Avantageusement selon la présente invention, le premier système de solvants comprend en outre de l'hexaméthyldisiloxane (HMDS), typiquement à une teneur comprise entre 0,1 et 49% en volume, par rapport au volume total du premier système de solvant.

Les numéros CAS du HMDS est le 107-46-0.

Selon un mode de réalisation particulier, le premier système de solvant peut comprendre :

- une teneur en solvant choisi parmi les solvants comprenant de 5 à 8 atomes de carbone, et un ou deux atomes d'oxygène sous forme soit de fonction éther, soit de fonction cétone, soit de fonction ester, choisi parmi les méthylcétones, notamment le MIBK, le 2-heptanone, les propionates, notamment l'éthyl propionate, le n-butyl propionate, l'isoamyl propionate, les propyléthers, notamment le DIPE, et leurs mélanges, d'au moins 50% en volume par rapport au volume total du premier système de solvants, et

- du HMDS, notamment en une teneur allant de 0,1 à 49%, tout particulièrement de 0,5 à 30%, voire de 1 à 20%, et tout particulièrement de 5 à 10 % en volume par rapport au volume total du premier système de solvants.

Le premier système de solvants peut comprendre :

- une teneur en solvant choisi parmi les solvants comprenant de 5 à 8 atomes de carbone, et un ou deux atomes d'oxygène sous forme de fonction éther, cétone ou ester, choisi parmi les méthylcétones, notamment le MIBK, le 2-heptanone, les propionates, notamment l'éthyl propionate, le n-butyl propionate, l'isoamyl propionate, les propyléthers, notamment le DIPE, et leurs mélanges, d'au moins 60%, notamment au moins 75%, en particulier au moins 90%, et plus particulièrement au moins 95% en volume par rapport au volume total du premier système de solvants, et

- du HMDS, notamment en une teneur allant de 0,1 à 40%, tout particulièrement de 0,5 à 25%, voire de 1 à 20%, et tout particulièrement de 5 à 10 % en volume par rapport au volume total du premier système de solvant.

En particulier, le premier système de solvants est constitué :

- de solvant comprenant de 5 à 8 atomes de carbone, et un ou deux atomes d'oxygène sous forme de fonction éther, cétone ou ester, choisi parmi les méthylcétones, notamment le MIBK, le 2-heptanone, les propionates, notamment l'éthyl propionate, le n-butyl propionate, l'isoamyl propionate, les propyléthers, notamment le DIPE, ou d'un mélange de ceux-ci, avantageusement à une teneur d'au moins 50% en volume, par rapport au volume total du premier système de solvant et

- de HMDS, notamment en une teneur allant de 0,1 à 49%, tout particulièrement de 0,5 à 30%, voire de 1 à 20%, et tout particulièrement de 5 à 10 % en volume par rapport au volume total du premier système de solvant.

Le premier système de solvants peut comprendre :

- une teneur en un solvant choisi parmi les solvants comprenant de 5 à 8 atomes de carbone, et un ou deux atomes d'oxygène sous forme de fonction éther, cétone ou ester, choisi parmi les méthylcétones, notamment le MIBK, le 2-heptanone, les propionates, notamment l'éthyl propionate, le n-butyl propionate, l'isoamyl propionate, et les propyléthers, notamment le DIPE, d'au moins 60%, notamment au moins 75%, en particulier au moins 90%, plus particulièrement au moins 95%, encore plus particulièrement au moins 99% en volume par rapport au volume total du premier système de solvants, et
- du HMDS, notamment en une teneur allant de 0,1 à 40%, tout particulièrement de 0,5 à 25%, voire de 1 à 20%, et tout particulièrement de 5 à 10% en volume par rapport au volume total du premier système de solvant.

Selon une variante, le premier système de solvants est constitué par :

- un solvant comprenant de 5 à 8 atomes de carbone, et un ou deux atomes d'oxygène sous forme de fonction éther, cétone ou ester, choisi parmi les méthylcétones, notamment le MIBK, le 2-heptanone, les propionates, notamment l'éthyl propionate, le n-butyl propionate, l'isoamyl propionate, et les propyléthers, notamment le DIPE, avantageusement à une teneur d'au moins 50% en volume, par rapport au volume total du premier système de solvant, et
- du HMDS, notamment en une teneur allant de 0,1 à 49%, tout particulièrement de 0,5 à 25%, voire de 1 à 20%, et tout particulièrement de 5 à 10% en volume par rapport au volume total du premier système de solvant.

Selon un mode de réalisation particulier, le premier système de solvants comprend une teneur en solvant(s) CMR, en particulier présent(s) sur la liste CMR UE1, UE2, et/ou US, inférieure ou égale à 10%, notamment inférieure ou égale à 5%, en particulier inférieure ou égale à 2%, tout particulièrement inférieure ou égale à 1%, encore plus particulièrement inférieure ou égale à 0,5%, voire inférieure ou égale à 0,1% en volume par rapport au volume total du premier système de solvants.

Encore plus particulièrement le premier système de solvants est dépourvu de solvants présents sur la liste CMR UE1, UE2 et/ou US, et tout particulièrement d'hexane et de dichloroéthane.

Les solvants utilisés dans le premier système de solvants présentent une pureté d'au moins 90%, notamment d'au moins 95%, en particulier d'au moins 98%, tout particulièrement d'au moins 99%, voire d'au moins 99,5%.

L'invention concerne également un procédé d'obtention d'une fraction insaponifiable, notamment totale ou partielle, comprenant au moins les étapes suivantes :

- extraction solide / liquide d'au moins une matière solide végétale ou d'un micro-organisme par un premier système de solvants comprenant une teneur en solvant choisi parmi les solvants comprenant entre 5 et 8 atomes de carbone et un ou deux atomes d'oxygène sous forme soit de fonction éther, soit de fonction cétone, soit de fonction ester, choisi parmi les méthylcétones, notamment le MIBK, le 2-heptanone, les propionates, notamment l'éthyl propionate, le n-butyl propionate, l'isoamyl propionate, les propyléthers, notamment le DIPE, et leurs mélanges, d'au moins 50% en volume par rapport au volume total du premier système de solvants,
- avantageusement récupération d'un extrait naturel enrichi en huile ou en beurre, en particulier sous forme de solution organique enrichie en huile ou beurre, voire récupération de l'huile ou du beurre, notamment ledit extrait naturel est enrichi en insaponifiable,
- transformation de ladite huile ou dudit beurre en solution hydro-alcoolique, via une étape de saponification,
- extraction de la solution hydro-alcoolique dans laquelle la fraction grasse est séparée de la fraction insaponifiable par une extraction liquide / liquide, et
- récupération de la fraction insaponifiable, notamment partielle ou totale.

Par « fraction totale », on entend au sens de la présente invention le fait que cette fraction comprend toutes les familles de substances composant l'insaponifiable présentes dans l'huile ou dans le beurre végétal ou dans le micro-organisme considéré.

Par « fraction partielle », on entend au sens de la présente invention le fait que cette fraction comprend au moins une des familles de substances composant l'insaponifiable présente dans l'huile ou dans le beurre végétal ou dans le micro-organisme considéré.

Le premier système de solvants est tel que défini dans le cas du procédé d'extraction solide / liquide d'un extrait végétal.

De manière particulièrement avantageuse, les solvants d'extraction solide / liquide selon l'invention comprennent entre 5 et 8 atomes de carbone, et présentent ainsi de petites chaînes carbonées ; ils ont donc l'avantage d'être suffisamment lipophiles pour extraire une huile et/ou un beurre et les fractions insaponifiables qu'ils contiennent et de pouvoir être ensuite facilement séparés de l'extrait obtenu.

La transformation dudit extrait naturel, notamment de ladite huile ou dudit beurre, en solution hydro-alcoolique peut être effectuée dans un système de solvants classique.

Selon une variante particulière, la transformation dudit extrait naturel, notamment de ladite huile ou dudit beurre, en solution hydro-alcoolique peut être effectuée dans un deuxième système de solvants comprenant, voire consistant en, au moins un solvant du premier système de solvants.

Plus particulièrement, la transformation peut être effectuée sans purification totale dudit extrait naturel, notamment de l'huile ou du beurre. En particulier, la transformation est effectuée directement sur la base de la solution organique enrichie dudit extrait naturel, notamment en huile ou en beurre, notamment comprenant au moins 2% en masse, en particulier au moins 5 % en masse, voire au moins 10% en masse d'huile ou de beurre par rapport à la masse totale de la solution organique enrichie en huile ou en beurre.

Selon une première variante, la transformation est réalisée sur une fraction, notamment en partie évaporée, à laquelle est ajoutée moins de 50% en masse d'autres solvants, voire il n'est pas ajouté d'autres solvants.

Selon une autre variante au moins 10% d'au moins un autre solvant, comme des alcools en C2 à C4, notamment l'éthanol, le n-propanol, l'iso-propanol, le butanol, en particulier le n-butanol, le méthyltétrahydrofurane (MeTHF) et leurs mélanges, peuvent être ajoutés à la solution organique enrichie en huile ou en beurre, notamment évaporée.

Dans le cas où l'extraction de la solution hydro-alcoolique est effectuée par une extraction liquide / liquide, celle-ci peut être faite avec un troisième système de solvants défini de la même manière que le premier système de solvants. Et en particulier, ce troisième système de solvants comprend, voire consiste en, les mêmes solvants que ceux utilisés dans le premier et/ou le deuxième système de solvants.

De manière générale, le procédé d'extraction solide/liquide selon l'invention peut être plus économique, plus direct, plus amical pour l'environnement nécessitant une quantité de solvant organique plus faible, plus facile à mettre en oeuvre, plus rapide, générant des conditions moins toxiques, permettant l'obtention d'huiles ou de beurres, notamment présentant une teneur importante en insaponifiable, avec un rendement et/ou une sélectivité au moins comparables, voire supérieurs, aux procédés déjà existants.

La partie saponification et extraction de l'insaponifiable peut notamment être effectuée selon les procédures décrites dans EP 1 246 633.

La matière solide végétale ou le micro-organisme utilisé dans les présents procédés peut provenir du soja, du colza, du maïs, du tournesol, du sésame, du lupin, du coton, de la noix de coco, d'olive, d'avocat, de cacao, d'illipé, de karité, de palmiste, d'arachide, de coprah, de lin, de ricin, de pépins de raisin, de pépins de courge, de pépins de cassis, de pépins de melon, de pépins de tomate, de pépins de citrouille, d'amande, de noisette, de noix, d'onagre, de bourrache, de carthame, de cameline, d'oeillette, de macro-algues, de micro-algues, telles que les Haematocococcus, Dunaliella, Spirulina, Chorella, et/ou de micro-organismes, notamment marin, d'eau douce ou terrestre, en particulier de levures, de moisissures, et plus particulièrement, de bactéries, et leurs mélanges.

Typiquement, les teneurs de fraction insaponifiable obtenues s'échelonnent de 2 à 10% dans l'huile d'avocat, sont d'environ 0,5% dans l'huile de coco, d'environ 1% dans l'huile de soja et d'environ 1 % dans l'huile d'olive.

L'Homme de l'Art connaît les procédés à mettre en oeuvre pour extraire la fraction insaponifiable d'une huile ou d'un beurre végétal ou d'un micro-organisme et sait les appliquer à la partie transformation, extraction et/ou récupération de l'insaponifiable de la présente invention.

Parmi l'art antérieur portant sur cette partie, on peut citer en particulier le procédé de préparation d'insaponifiable d'huile d'avocat tel que décrit et revendiqué dans le brevet FR 2 678 632.

Ainsi, l'insaponifiable d'huile d'avocat peut être préparé à partir du fruit préalablement traité thermiquement, avant l'extraction de l'huile et la saponification, comme décrit dans le brevet FR 2 678 632.

Ce traitement thermique consiste en un séchage contrôlé du fruit, frais de préférence, pendant au moins quatre heures, avantageusement au moins 10 heures, de préférence entre environ 24 et environ 48 heures, à une température de préférence d'au moins environ 80°C et de préférence comprise entre environ 80 et environ 120°C.

On peut également citer le procédé de préparation d'insaponifiable d'huile de soja, obtenu à partir d'un concentrât d'insaponifiable d'huile de soja.

Ledit concentrât d'insaponifiable peut être préparé par distillation moléculaire selon un procédé tel que décrit pour l'huile de lupin dans la demande de brevet FR 2 762 512, mais adapté à l'huile de soja.

Dans ce procédé, l'huile de soja est distillée dans un distillateur moléculaire de type centrifuge ou à film raclé, à une température comprise entre environ 210 et 250°C et sous un vide poussé, compris entre 0,01 et 0,001 millimètres de mercure (soit 0,13 à 1,3 Pa).

Le distillat obtenu présente une teneur en insaponifiable comprise entre 5 et 40% en masse et constitue donc un concentrât d'insaponifiable d'huile de soja.

Le concentrât est ensuite saponifié par une base telle que la potasse ou la soude en milieu polaire, notamment alcoolique, de préférence de l'éthanol, du n-propanol, de l'iso-propanol, du butanol, en particulier du n-butanol, du méthyltétrahydrofurane (MeTHF), ou un mélange de ceux-ci, puis il est soumis à une ou plusieurs extractions par le troisième système de solvants.

La solution d'extraction obtenue est de préférence ensuite centrifugée, filtrée puis lavée à l'eau pour éliminer les traces résiduelles d'alcalinité.

Le solvant d'extraction est évaporé soigneusement pour récupérer l'insaponifiable. On peut également bien entendu prévoir des opérations supplémentaires connues de l'homme du métier, telles qu'une étape de désodorisation.

Enfin, avant sa saponification, l'extrait naturel, notamment l'huile ou le beurre, peut être préalablement enrichie en insaponifiable en séparant une majorité des constituants de l'insaponifiable que l'on récupère dans un concentrât. Différentes méthodes peuvent être utilisées : cristallisation par le froid, extraction liquide / liquide, ou encore distillation moléculaire.

La concentration préalable de l'huile ou du beurre en insaponifiable permet de diminuer les volumes d'extrait naturel, notamment d'huile ou de beurre, à saponifier.

La distillation moléculaire est particulièrement préférée, étant réalisée de préférence à une température comprise entre environ 180 et environ 230°C en maintenant une pression comprise entre $10^{-3}$ et $10^{-2}$ mm Hg et de préférence de l'ordre de $10^{-3}$ mm Hg.

La concentration en insaponifiable du distillat peut atteindre 60% en masse par rapport à la masse totale.

Tout particulièrement, la présente invention concerne un procédé tel que décrit dans la présente description dans lequel l'insaponifiable obtenu est choisi parmi un insaponifiable de soja, avantageusement enrichi en stérols, tocophérols, et/ou squalène, un insaponifiable d'avocat, notamment un insaponifiable d'avocat enrichi en fraction furanique et/ou un insaponifiable d'avocat enrichi en fraction stérolique et/ou un insaponifiable d'avocat enrichi en composés trihydroxylés.

La présente description concerne encore une huile ou un beurre dépourvu de solvants classés dans la liste CMR UE1, liste CMR UE2 et/ou US, en particulier ladite huile ou ledit beurre est susceptible d'être obtenu, ou directement obtenu, par le procédé selon la présente invention.

La présente description divulgue encore une fraction insaponifiable, notamment partielle ou totale, dépourvue de solvants classés dans la liste CMR UE1, UE2 et/ou US, en particulier ladite fraction est obtenue par le procédé d'extraction selon la présente invention.

La présente description concerne encore l'utilisation de cette fraction, de cet extrait naturel, de ce beurre ou de cette huile pour la préparation d'une composition, notamment pharmaceutique, alimentaire et/ou cosmétique, ou encore d'un complément alimentaire.

La présente description divulgue également la fraction insaponifiable, notamment partielle ou totale, l'huile ou le beure, dépourvu(e) de solvants classés dans la liste CMR UE1, UE2 et/ou US, tel(le) que décrit(e) ci-dessus, pour son utilisation comme médicament, comme dispositif médical, comme agent dermatologique, comme agent cosmétique, ou comme nutraceutique, à visée humaine ou animale, avantageusement dans la prévention et/ou le traitement des troubles du tissu conjonctif tels que l'arthrose, des pathologies articulaires telles que les rhumatismes, des maladies parodontales, telles que la gingivite ou la parodontite, ou encore dans la prévention et/ou le traitement des troubles du derme et/ou de l'hypoderme tels que le vieillissement cutané, les vergetures et la cellulite, ou encore des troubles de la barrière épidermique tels que les inflammations cutanées, l'eczéma atopique et les dermatites irritatives et/ou inflammatoires.

La présente description divulgue également une composition, notamment alimentaire, cosmétique ou pharmaceutique, ou encore un complément alimentaire, comprenant au moins un extrait naturel, une huile, un beurre ou une fraction insaponifiable d'au moins un extrait naturel, d'une huile ou d'un beurre végétal ou d'un micro-organisme, ledit extrait, huile, beurre ou fraction étant dépourvu de solvants classés dans la liste CMR UE1, UE2 et/ou US et/ou ledit extrait, huile, beurre ou fraction est susceptible d'être obtenu, ou directement obtenu, par le procédé selon l'invention, et ladite composition comprenant éventuellement un excipient, en particulier cosmétiquement, alimentairement ou pharmaceutiquement acceptable.

En particulier, la présente description concerne une composition, notamment pharmaceutique, alimentaire ou cosmétique, ou encore un complément alimentaire, comprenant au moins un insaponifiable, en particulier un insaponifiable de soja, un insaponifiable d'avocat, tout particulièrement un insaponifiable d'avocat riche en fraction furanique et/ou un insaponifiable d'avocat riche en fraction stérolique, susceptible d'être obtenu ou directement obtenu par le procédé selon l'invention.

Les compositions pharmaceutiques, alimentaires, ou compléments alimentaires peuvent être destinés à la prévention et/ou au traitement de troubles du tissu conjonctif, notamment de l'arthrose, des parodontopathies, du vieillissement cutané et/ou des inflammations cutanées.

Les compositions pharmaceutiques ou cosmétiques selon la description peuvent être destinées à la prévention et/ou au traitement des troubles cutanées de l'épiderme, du derme et/ou de l'hypoderme.

Par « dépourvu de solvants classés dans la liste CMR UE1, UE2 et/ou US », on entend au sens de la présente invention une teneur totale en solvants classés dans la liste CMR UE1, UE2 et/ou US inférieure à 10 ppm, notamment inférieure à 5 ppm, en particulier inférieure à 2 ppm, voire inférieure à 1 ppm.

La présente description vise encore un procédé de traitement cosmétique tel que l'on applique de façon topique la composition cosmétique selon l'invention.

La description concerne aussi un extrait végétal, une huile, un beurre, ou un insaponifiable d'une huile ou d'un beurre végétal ou d'un micro-organisme obtenu ou susceptible d'être obtenu selon la présente description pour son utilisation en tant que médicament, en particulier destiné à traiter ou à prévenir des troubles du tissu conjonctif, et notamment l'arthrose.

Selon encore un autre aspect, la description divulgue l'utilisation de HMDS, en une teneur allant de 0,1 à 49%, typiquement de 0,1 à 45%, en volume par rapport au volume total des solvants d'extraction ou systèmes d'extraction, dans un procédé d'extraction solide/liquide notamment d'insaponifiables, avantageusement à partir d'une matière solide végétale ou d'un micro-organisme.

Le HMDS peut être présent en une teneur allant de 0,5 à 25%, voire de 1 à 20%, et tout particulièrement de 5 à 10 % en volume par rapport au volume total de solvant.

En particulier ledit procédé comprend un système de solvant comprenant :

- au moins un solvant comprenant au moins 5 atomes de carbone, voire de 5 à 8 atomes de carbone, et un ou deux atomes d'oxygène sous forme de fonction éther, cétone ou ester, en particulier au moins un solvant choisi parmi

  ◦ les méthylcétones, notamment le MIBK, le 2-heptanone,
  ◦ les propionates, notamment l'éthyl propionate, le n-butyl propionate, l'isoamyl propionate,
  ◦ les propyléthers, notamment le DIPE,
  ◦ un mélange de ceux-ci, ou

- au moins un solvant choisi parmi

  ◦ les solvants aromatiques fluorés, notamment le trifluorotoluène (BTF) et l'hexafluorobenzène (BHF),
  ◦ les tert-butyl éthers, notamment le 2-éthoxy-2-méthylpropane, encore appelé éthyl-tert-butyl-éther (ETBE), et le 2-méthoxy-2-méthylpropane, encore appelé méthyl-tert-butyl-éther (MTBE),
  ◦ les solvants comprenant au moins un atome de silicium, notamment l'hexaméthyldisiloxane (HMDS) et le tétraméthylsilane (TMS),
  ◦ le méthyl-tétrahydrofurane (MeTHF), et
  ◦ leurs mélanges, ou

- un mélange de solvants ci-dessus.

La teneur en ces solvants peut être telle que décrite ci-dessus.

Les numéros CAS de ces différents solvants sont les suivants BTF : 98-08-8 ; BHF : 392-56-3 ; ETBE : 637-92-3 ; MTBE : 1634-04-4 ; TMS : 75-76-3 ; et MeTHF : 96-47-9.

La présence de HMDS parmi les solvants d'extraction peut permettre d'affiner le profil de l'insaponifiable obtenu, d'améliorer le taux d'extraction en une ou plusieurs fractions, et/ou le rendement global d'extraction.

Le HMDS peut notamment permettre de moduler la capacité extractive du système de solvant. Ainsi, la présence de HMDS parmi les solvants d'extraction peut permettre d'améliorer le rendement en huile et/ou permettre d'améliorer la teneur en insaponifiable dans cette huile, affiner le profil de l'insaponifiable obtenu et/ou le taux d'extraction en une ou plusieurs fractions.

Par ailleurs, la présence de HMDS dans une teneur telle que définie ci-dessus dans un système de solvants peut permettre de diminuer la consommation de solvants, et/ou de temps d'extraction.

Ainsi, le HMDS peut être utilisé en tant qu'agent d'amélioration du rendement d'extraction, agent d'amélioration de la teneur en insaponifiable de l'extrait naturel, et/ou agent d'accélération.

Bien entendu, les différentes caractéristiques exposées dans la présente description peuvent être combinées entre elles.

A titre d'exemples illustrant la présente invention, les expérimentations suivantes ont été effectuées.

**EXEMPLES**

Dans tous les exemples, un essai de référence utilisant l'hexane a été réalisé.

Exemple 1 : Extraction à partir d'avocats déshydratés

Une extraction d'avocats déshydratés a été effectuée avec de l'hexane (référence) et avec les solvants suivants : 2-heptanone, MIBK et éthyl propionate.

De l'avocat déshydraté est broyé et introduit dans une cartouche de cellulose (30 à 40 g). L'extraction est réalisée dans un appareil de type Soxhlet (BUCHI B-811). Quatre extractions sont alors lancées en parallèle et correspondent chacune à 20 cycles d'extraction / siphonage. Une fois l'extraction finalisée, le solvant d'extraction est évaporé et le résidu désolvanté est pesé. Les rendements massiques sont ensuite comparés. Les résultats sont présentés dans le tableau 1.

Tableau 1

| Solvant d'extraction | Rendement massique d'extraction (% m/m) | Teneur en insaponifiable de l'extrait naturel (% m/m) | Augmentation de la teneur en insaponifiable de l'extrait naturel (%) |
|---|---|---|---|
| Hexane | 58,0 | 4,12 | 0 |
| 2-heptanone | 58,4 | 4,20 | +1,9 |
| MIBK | 58,4 | 4,49 | +9,0 |
| éthyl propionate | 58,5 | 4,51 | +9,5 |

Le rendement massique d'extraction correspond à :

$$R = 100 \text{ X (masse d'extrait / masse de matière solide mise en œuvre)}$$

La teneur en insaponifiable de l'extrait naturel est évaluée par chromatographie gazeuse.

L'augmentation de la teneur en insaponifiable de l'extrait naturel est calculée comme suit :

$$A = 100 \text{ X (teneur en insaponifiable de l'extrait naturel obtenu avec le solvant S - teneur en insaponifiable de l'extrait naturel obtenu avec l'hexane) / teneur en insaponifiable de l'extrait naturel obtenu avec l'hexane.}$$

Ces résultats montrent que les solvants selon la présente invention présentent des rendements d'extraction équivalents voire supérieurs à ceux de l'hexane. Dans tous les cas, la teneur en insaponifiable de l'extrait naturel obtenu est supérieure à celle de l'extrait naturel de référence obtenu avec l'hexane, l'augmentation variant de 1,9 à 9,5%.

Exemple 2 : Extraction à partir de coques de lupin broyée

Une extraction de coques de lupin broyée a été effectuée selon le mode opératoire de l'Exemple 1 et avec les solvants suivants : 2-heptanone, MIBK, éthyl propionate et isopropyléther. Les résultats sont présentés dans le tableau 2.

Tableau 2

| Solvant d'extraction | Rendement massique d'extraction (% m/m) | Augmentation du rendement massique d'extraction (% m/m) | Teneur en insaponifiable de l'extrait naturel (% m/m) | Taux d'extraction en insaponifiable pour 100g de matière solide (%) |
|---|---|---|---|---|
| Hexane | 0,93 | 0 | 29,1 | 0,27 |
| 2-heptanone | 1,43 | 53,8 | 18,2 | 0,26 |
| MIBK | 1,17 | 25,8 | 25,3 | 0,30 |
| éthyl propionate | 1,03 | 10,8 | 34,0 | 0,35 |
| isopropyléther | 0,99 | 6,5 | 19,4 | 0,19 |

Le rendement massique d'extraction correspond à :

$$R = 100 \text{ X (masse d'extrait / masse de matière solide mise en œuvre)}$$

L'augmentation du rendement massique d'extraction est calculée comme suit :

A' = 100 X (rendement massique d'extraction obtenu avec le solvant S - rendement massique d'extraction obtenu avec l'hexane) / rendement massique d'extraction obtenu avec l'hexane

La teneur en insaponifiable de l'extrait naturel est évaluée par chromatographie gazeuse.

Le taux d'extraction en insaponifiable pour 100g de matière solide est calculé comme suit :

$$T = \text{(rendement massique d'extraction x teneur en insaponifiable)}/100$$

Ces résultats montrent que les solvants selon la présente invention permettent d'améliorer le rendement massique d'extraction par rapport à celui de l'hexane, l'augmentation du rendement massique d'extraction allant de 6,5% avec le DIPE à 53,8% avec le 2-heptanone.

A l'exception du DIPE, les taux d'extraction en insaponifiable pour 100g de matière solide mis en oeuvre sont équivalents ou supérieurs à ceux obtenus avec l'hexane.

Exemple 3 : Extraction à partir de farine de graines de lupin

Une extraction de farine de graines de lupin a été effectuée avec de l'hexane (référence) et avec du MIBK.

60g de farine de graines de lupin sont introduits dans une cartouche de cellulose. L'extraction est réalisée dans un appareil de type Soxhlet (BUCHI B-811). Quatre extractions, deux par solvant, sont alors lancées en parallèle ; chacune correspond à 20 cycles d'extraction / siphonage. Une fois l'extraction finalisée, le solvant d'extraction est évaporé et le résidu désolvanté est pesé. Les rendements massiques sont ensuite comparés. La composition des huile en insaponifiable est ensuite analysée. Les résultats moyennés sont présentés dans le tableau 3.

Tableau 3

| Solvant d'extraction | Rendement massique d'extraction (% m/m) | Teneur en caroténoïdes de l'extrait naturel (mg/100g d'huile) | Augmentation de la teneur en caroténoide de l'extrait naturel (%) |
|---|---|---|---|
| Hexane | 12,5% | 24,9 | 0 |
| MIBK | 13,0% | 37,8 | +51,8% |

Le MIBK, solvant non CMR, permet d'augmenter le rendement d'extraction en huile de 0,5%. Le MIBK, en plus d'une faible toxicité, présente l'avantage d'augmenter le taux d'extraction en une fraction particulière d'insaponifiable, les caroténoïdes, de près de 52% massique, mettant en avant la faible dégradation de cette famille de composés au cours de l'étape d'extraction.

**Revendications**

1. Procédé d'extraction solide / liquide d'un extrait naturel contenu dans au moins une matière solide végétale ou un micro-organisme comprenant au moins les étapes suivantes :

- extraction solide / liquide d'au moins une matière solide végétale ou d'un micro-organisme par un premier système de solvants comprenant une teneur en solvant choisi parmi les solvants comprenant entre 5 et 8 atomes de carbone et un ou deux atomes d'oxygène sous forme soit de fonction éther, soit de fonction cétone, soit de fonction ester, d'au moins 50% en volume par rapport au volume total du premier système de solvant, lesdits solvants étant choisis parmi les méthylcétones, notamment le méthyl isobutyl cétone (MIBK), le 2-heptanone, les propionates, notamment l'éthyl propionate, le n-butyl propionate, l'isoamyl propionate, les propyléthers, notamment le diisopropyl éther (DIPE), et leurs mélanges,

- récupération d'un extrait naturel comprenant de l'huile ou du beurre, en particulier enrichi en insaponifiable.

2. Procédé d'obtention d'une fraction insaponifiable totale ou partielle, comprenant au moins les étapes suivantes :

- extraction solide / liquide d'au moins une matière solide végétale ou d'un micro-organisme par un premier système de solvants comprenant une teneur en solvant choisi parmi les solvants comprenant entre 5 et 8 atomes de carbone et un ou deux atomes d'oxygène sous forme soit de fonction éther, soit de fonction cétone, soit de fonction ester, d'au moins 50% en volume par rapport au volume total du premier système de solvants, lesdits solvants étant choisis parmi les méthylcétones, notamment le méthyl isobutyl cétone (MIBK), le 2-heptanone, les propionates, notamment l'éthyl propionate, le n-butyl propionate, l'isoamyl propionate, les propyléthers, notamment le diisopropyl éther (DIPE), et leurs mélanges,
- récupération d'un extrait naturel enrichi en huile ou en beurre, en particulier sous la forme d'une solution organique enrichie en huile ou en beurre,
- transformation dudit extrait naturel en solution hydro-alcoolique, via une étape de saponification,
- extraction de la solution hydro-alcoolique dans laquelle la fraction grasse est séparée de la fraction insaponifiable par une extraction liquide / liquide, et
- récupération de la fraction insaponifiable.

3. Procédé selon la revendication 1 ou 2, **caractérisé en ce que** le premier système de solvant présente une densité inférieure à 1.

4. Procédé selon l'une quelconque des revendications 1 à 3, **caractérisé en ce que** le premier système de solvant comprend une teneur en solvant comprenant entre 5 et 8 atomes de carbone et un ou deux atomes d'oxygène d'au moins 60%, notamment au moins 75%, en particulier au moins 90%, plus particulièrement au moins 95%, encore plus particulièrement au moins 99% en volume, par rapport au volume total du premier système de solvants.

5. Procédé selon l'une quelconque des revendications 1 à 4, **caractérisé en ce que** le premier système de solvants comprend une teneur en un seul solvant choisi parmi les méthylcétones, notamment le méthyl isobutyl cétone (MIBK), le 2-heptanone, les propionates, notamment l'éthyl propionate, le n-butyl propionate, l'isoamyl propionate, et les propyléthers, notamment le diisopropyl éther (DIPE), d'au moins 60%, notamment au moins 75%, en particulier au moins 90%, plus particulièrement au moins 95%, encore plus particulièrement au moins 99%, en volume par rapport au volume total du premier système de solvants.

6. Procédé selon l'une quelconque des revendications 1 à 5, **caractérisé en ce que** le premier système de solvant comprend en outre du hexaméthyldisiloxane (HMDS), avantageusement à une teneur comprise entre 0,1 et 49% en volume par rapport au volume total du premier système de solvants.

7. Procédé selon l'une quelconque des revendications 1 à 6, **caractérisé en ce que** le premier système de solvant comprend une teneur en solvant(s) CMR, en particulier présent(s) sur la liste CMR UE1, UE2, et/ou US, inférieure ou égale à 10%, notamment inférieure ou égale à 5%, en particulier inférieure ou égale à 2%, tout particulièrement inférieure ou égale à 1%, encore plus particulièrement inférieure ou égale à 0,5%, voire inférieure ou égale à 0,1%, en volume par rapport au volume total du premier système de solvants.

8. Procédé selon l'une quelconque des revendications 2 à 7, **caractérisé en ce que** la transformation dudit extrait naturel en solution hydro-alcoolique est effectuée dans un deuxième système de solvants comprenant une teneur en solvant choisi parmi les alcools de C2 à C4, et notamment l'éthanol, le n-propanol, l'iso-propanol, le butanol, en particulier le n-butanol, le méthyltétrahydrofurane (MeTHF) et leurs mélanges, d'au moins 10% en volume par rapport au volume total du deuxième système de solvants, ledit deuxième système de solvants pouvant être ajouté à la solution organique enrichie en huile ou en beurre, notamment évaporée.

9. Procédé selon l'une quelconque des revendications 2 à 7, **caractérisé en ce que** la transformation dudit extrait naturel en solution hydro-alcoolique est effectuée dans un deuxième système de solvants comprenant, voire consistant en, au moins un solvant du premier système de solvants.

10. Procédé selon l'une quelconque des revendications 2 à 9, **caractérisé en ce que** la transformation est effectuée directement sur la base de la solution organique enrichie en huile ou en beurre, notamment comprenant au moins 2% en masse, en particulier au moins 5 % en masse, voire au moins 10% en masse d'huile ou de beurre par rapport à la masse totale de la solution organique enrichie en huile ou en beurre.

**11.** Procédé selon la revendication 10, **caractérisé en ce que** la transformation est réalisée sur une fraction, notamment en partie évaporée, à laquelle il est ajouté moins de 50% en masse d'autres solvants, voire il n'est pas ajouté d'autres solvants.

**12.** Procédé selon l'une quelconque des revendications 2 à 11, **caractérisé en ce que** l'extraction de la solution hydro-alcoolique est effectuée par une extraction liquide / liquide faite avec un troisième système de solvants comprenant en tout ou partie les mêmes solvants que ceux utilisés dans le premier et/ou le deuxième système de solvants.

**13.** Procédé selon l'une quelconque des revendications 1 à 12, caractérisé en ce l'insaponifiable obtenu est choisi parmi un insaponifiable de soja, un insaponifiable d'avocat, notamment un insaponifiable d'avocat enrichi en fraction furanique et/ou un insaponifiable d'avocat enrichi en fraction stérolique, et plus particulièrement un mélange d'insaponifiables d'avocat et de soja (ASU).

**Patentansprüche**

**1.** Verfahren zur Fest/Flüssig-Extraktion eines natürlichen Extrakts, der in wenigstens einem pflanzlichen Feststoff oder einem Mikroorganismus enthaltenen ist, umfassend wenigstens die folgenden Schritte:

- Fest/Flüssig-Extraktion von wenigstens einem pflanzlichen Feststoff oder einem Mikroorganismus durch ein erstes Lösungsmittelsystem, umfassend einen Gehalt an Lösungsmittel, ausgewählt aus den Lösungsmitteln, die zwischen 5 und 8 Kohlenstoffatome und ein oder zwei Sauerstoffatome in Form entweder einer Etherfunktion oder Ketonfunktion oder Esterfunktion umfassen, von wenigstens 50 Vol.-% bezogen auf das Gesamtvolumen des ersten Lösungsmittelsystems, wobei die Lösungsmittel aus den Methylketonen, insbesondere dem Methylisobutylketon (MIBK), dem 2-Heptanon, den Propionaten, insbesondere dem Ethylpropionat, dem n-Butylpropionat, dem Isoamylpropionat, den Propylethern, insbesondere dem Diisopropylether (DIPE), und deren Mischungen ausgewählt sind,
- Gewinnen eines natürlichen Extrakts, der Öl oder Butter, speziell an unverseifbaren Anteilen angereichert, umfasst.

**2.** Verfahren zum Erhalten einer vollständig oder teilweise unverseifbaren Fraktion, umfassend wenigstens die folgenden Schritte:

- Fest/Flüssig-Extraktion von wenigstens einem pflanzlichen Feststoff oder einem Mikroorganismus durch ein erstes Lösungsmittelsystem, umfassend einen Gehalt an Lösungsmittel, ausgewählt aus den Lösungsmitteln, die zwischen 5 und 8 Kohlenstoffatome und ein oder zwei Sauerstoffatome in Form entweder einer Etherfunktion oder Ketonfunktion oder Esterfunktion umfassen, von wenigstens 50 Vol.-% bezogen auf das Gesamtvolumen des ersten Lösungsmittelsystems, wobei die Lösungsmittel aus den Methylketonen, insbesondere dem Methylisobutylketon (MIBK), dem 2-Heptanon, den Propionaten, insbesondere dem Ethylpropionat, dem n-Butylpropionat, dem Isoamylpropionat, den Propylethern, insbesondere dem Diisopropylether (DIPE), und deren Mischungen ausgewählt sind,
- Gewinnen eines natürlichen, an Öl oder an Butter angereicherten Extrakts, speziell in der Form einer organischen, an Öl oder an Butter angereicherten Lösung,
- Umwandeln des natürlichen Extrakts in wässrig-alkoholische Lösung über einen Schritt der Verseifung,
- Extraktion der wässrig-alkoholischen Lösung, in der die Fettfraktion durch eine Flüssig/Flüssig-Extraktion von der unverseifbaren Fraktion abgetrennt wird, und
- Gewinnen der unverseifbaren Fraktion.

**3.** Verfahren nach Anspruch 1 oder 2, **dadurch gekennzeichnet, dass** das erste Lösungsmittelsystem eine Dichte von kleiner als 1 aufweist.

**4.** Verfahren nach einem der Ansprüche 1 bis 3, **dadurch gekennzeichnet, dass** das erste Lösungsmittelsystem einen Gehalt an Lösungsmittel, das zwischen 5 und 8 Kohlenstoffatome und ein oder zwei Sauerstoffatome umfasst, von wenigstens 60 Vol.-%, insbesondere wenigstens 75 Vol.-%, speziell wenigstens 90 Vol.-%, spezieller wenigstens 95 Vol.-%, noch spezieller wenigstens 99 Vol.-% bezogen auf das Gesamtvolumen des ersten Lösungsmittelsystems umfasst.

**5.** Verfahren nach einem der Ansprüche 1 bis 4, **dadurch gekennzeichnet, dass** das erste Lösungsmittelsystem einen Gehalt an einem einzigen Lösungsmittel, ausgewählt aus den Methylketonen, insbesondere dem Methylisobutylketon (MIBK), dem 2-Heptanon, den Propionaten, insbesondere dem Ethylpropionat, dem n-Butylpropionat, dem Isoamylpropionat, und den Propylethern, insbesondere dem Diisopropylether (DIPE), von wenigstens 60 Vol.-%, insbesondere wenigstens 75 Vol.-%, speziell wenigstens 90 Vol.-%, spezieller wenigstens 95 Vol.-%, noch spezieller wenigstens 99 Vol.-% bezogen auf das Gesamtvolumen des ersten Lösungsmittelsystems umfasst.

6. Verfahren nach einem der Ansprüche 1 bis 5, **dadurch gekennzeichnet, dass** das erste Lösungsmittelsystem weiter Hexamethyldisiloxan (HMDS), vorteilhafterweise in einem Gehalt im Bereich zwischen 0,1 und 49 Vol.-% bezogen auf das Gesamtvolumen des ersten Lösungsmittelsystems umfasst.

7. Verfahren nach einem der Ansprüche 1 bis 6, **dadurch gekennzeichnet, dass** das erste Lösungsmittelsystem einen Gehalt an CMR-Lösungsmittel(n), das/die insbesondere auf der CMR UE1-, UE2- und/oder US-Liste steht/stehen, von kleiner oder gleich 10 Vol.-%, insbesondere kleiner oder gleich 5 Vol.-%, speziell kleiner oder gleich 2 Vol.-%, ganz speziell kleiner oder gleich 1 Vol.-%, noch spezieller kleiner oder gleich 0,5 Vol.-%, ja sogar kleiner oder gleich 0,1 Vol.-% bezogen auf das Gesamtvolumen des ersten Lösungsmittelsystems umfasst.

8. Verfahren nach einem der Ansprüche 2 bis 7, **dadurch gekennzeichnet, dass** die Umwandlung des natürlichen Extrakts in wässrig-alkoholische Lösung in einem zweiten Lösungsmittelsystem durchgeführt wird, das einen Gehalt an Lösungsmittel, ausgewählt aus den C2- bis C4-Alkoholen, und insbesondere dem Ethanol, dem n-Propanol, dem Isopropanol, dem Butanol, insbesondere dem n-Butanol, dem Methyltetrahydrofuran (MeTHF) und deren Mischungen, von wenigstens 10 Vol.-% bezogen auf das Gesamtvolumen des zweiten Lösungsmittelsystems umfasst, wobei das zweite Lösungsmittelsystem der organischen, an Öl oder an Butter angereicherten, insbesondere verdampften Lösung zugefügt werden kann.

9. Verfahren nach einem der Ansprüche 2 bis 7, **dadurch gekennzeichnet, dass** die Umwandlung des natürlichen Extrakts in wässrig-alkoholische Lösung in einem zweiten Lösungsmittelsystem durchgeführt wird, das wenigstens ein Lösungsmittel des ersten Lösungsmittelsystems umfasst, ja sogar daraus besteht.

10. Verfahren nach einem der Ansprüche 2 bis 9, **dadurch gekennzeichnet, dass** die Umwandlung direkt auf der Grundlage der organischen, an Öl oder an Butter angereicherten Lösung durchgeführt wird, die insbesondere wenigstens 2 Gew.-%, speziell wenigstens 5 Gew.-%, ja sogar wenigstens 10 Gew.-% Öl oder Butter bezogen auf das Gesamtgewicht der organischen, an Öl oder an Butter angereicherten Lösung umfasst.

11. Verfahren nach Anspruch 10, **dadurch gekennzeichnet, dass** die Umwandlung an einer Fraktion, insbesondere zum Teil verdampften, ausgeführt wird, der weniger als 50 Gew.-% an anderen Lösungsmitteln zugefügt, ja sogar keine anderen Lösungsmittel zugefügt werden.

12. Verfahren nach einem der Ansprüche 2 bis 11, **dadurch gekennzeichnet, dass** die Extraktion der wässrig-alkoholischen Lösung durch eine Flüssig/Flüssig-Extraktion durchgeführt wird, die mit einem dritten Lösungsmittelsystem erfolgt, das vollständig oder teilweise die gleichen Lösungsmittel wie die, die in dem ersten und/oder dem zweiten Lösungsmittelsystem verwendet werden, umfasst.

13. Verfahren nach einem der Ansprüche 1 bis 12, **dadurch gekennzeichnet, dass** der erhaltene unverseifbare Anteil aus einem unverseifbaren Sojaanteil, einem unverseifbaren Avocadoanteil, insbesondere einem an Furanfraktion angereicherten unverseifbaren Avocadoanteil und/oder einem an Sterinfraktion angereicherten unverseifbaren Avocadoanteil, und spezieller einer Mischung aus unverseifbaren Avocado- und Sojaanteilen (ASU) ausgewählt ist.

## Claims

1. Process for the solid/liquid extraction of a natural extract contained in at least a plant-based solid material or a micro-organism comprising at least the following steps:

   - solid/liquid extraction of at least one plant-based solid material or a micro-organism by a first solvent system comprising a solvent content chosen from the solvents comprising between 5 and 8 carbon atoms and one or two oxygen atoms in the form of either an ether functional group, ketone functional group, or ester functional group, of at least 50% by volume with respect to the total volume of the first solvent system, said solvents being chosen from methylketones, particularly methyl isobutyl ketone (MIBK), 2-heptanone, propionates, particularly ethyl propionate, n-butyl propionate, isoamyl propionate, propylethers, particularly diisopropyl ether (DIPE), and mixtures thereof,
- retrieval of a natural extract comprising oil or butter, in particular enriched with unsaponifiable matter.

2. Process for obtaining a total or partial unsaponifiable fraction, comprising at least the following steps:

- solid/liquid extraction of at least a plant-based solid material or a micro-organism by a first solvent system comprising a solvent content chosen from the solvents comprising between 5 and 8 carbon atoms and one or two oxygen atoms in the form of either an ether functional group, ketone functional group, or ester functional group, of at least 50% by volume with respect to the total volume of the first solvent system, said solvents being chosen from methylketones, particularly methyl isobutyl ketone (MIBK), 2-heptanone, propionates, particularly ethyl propionate, n-butyl propionate, isoamyl propionate, propylethers, particularly diisopropyl ether (DIPE), and mixtures thereof,
- retrieval of a natural extract enriched with oil or with butter, in particular in the form of an organic solution enriched with oil or with butter,
- conversion of said natural extract into hydro-alcoholic solution, via a saponification step,
- extraction of the hydro-alcohol solution wherein the fat fraction is separated from the unsaponifiable fraction by liquid/liquid extraction, and
- retrieval of the unsaponifiable fraction.

3. Process according to claim 1 or 2, **characterised in that** the first solvent system has a density less than 1.

4. Process according to any one of claims 1 to 3, **characterised in that** the first solvent system comprises a solvent content comprising between 5 and 8 carbon atoms and one or two oxygen atoms of at least 60%, particularly at least 75%, in particular at least 90%, more particularly at least 95%, even more particularly at least 99% by volume, with respect to the total volume of the first solvent system.

5. Process according to any one of claims 1 to 4, **characterised in that** the first solvent system comprises a content of a single solvent chosen from methylketones, particularly methyl isobutyl ketone (MIBK), 2-heptanone, propionates, particularly ethyl propionate, n-butyl propionate, isoamyl propionate, propylethers, particularly diisopropyl ether (DIPE), of at least 60%, particularly at least 75%, in particular at least 90%, more particularly at least 95%, even more particularly at least 99% by volume, with respect to the total volume of the first solvent system.

6. Process according to any one of claims 1 to 5, **characterised in that** the first solvent further comprises hexamethyldisiloxane (HMDS), advantageously at a content between 0.1 and 49% by volume with respect to the total volume of the first solvent system.

7. Process according to any one of claims 1 to 6, **characterised in that** the first solvent comprises a content of CMR solvent(s), in particular contained in the EU1, EU2, and/or US CMR list, less than or equal to 10%, particularly less than or equal to 5%, in particular less than or equal to 2%, very particularly less than or equal to 1%, even more particularly less than or equal to 0.5%, or even less than or equal to 0.1%, by volume with respect to the total volume of the first solvent system.

8. Process according to any one of claims 2 to 7, **characterised in that** the conversion of said natural extract into hydro-alcoholic solution is performed in a second solvent system comprising a solvent content chosen from C2 to C4 alcohols, and particularly ethanol, n-propanol, iso-propanol, butanol, in particular n-butanol, methyltetrahydrofuran (MeTHF) and mixtures thereof, of at least 10% by volume with respect to the total volume of the second solvent system, said second solvent system being suitable for being added to the organic solution enriched with oil or with butter, particularly evaporated.

9. Process according to any one of claims 2 to 7, **characterised in that** the conversion of said natural extract into hydro-alcoholic solution is performed in a second solvent system comprising, or consisting of, at least one solvent of the first solvent system.

10. Process according to any one of claims 2 to 9, **characterised in that** the conversion is performed directly on the basis of the organic solution enriched with oil or with butter, particularly comprising at least 2% by mass, in particular at least 5% by mass, or at least 10% by mass of oil or butter with respect to the total mass of the organic solution enriched with oil or with butter.

**11.** Process according to claim 10, **characterised in that** the conversion is carried out on a fraction, particularly partially evaporated, to which less than 50% by mass of other solvents are added, or no other solvents are added.

**12.** Process according to any one of claims 2 to 11, **characterised in that** the extraction of the hydro-alcoholic solution is performed by a liquid/liquid extraction performed with a third solvent system comprising totally or partially the same solvents as those used in the first and/or the second solvent system.

**13.** Process according to any one of claims 1 to 12, **characterised in that** the unsaponifiable matter obtained is chosen from soybean unsaponifiable matter, avocado unsaponifiable matter, particularly avocado unsaponifiable matter enriched with furan fraction and/or avocado unsaponifiable matter enriched with sterol fraction, and more particularly a mixture of avocado and soybean unsaponifiable matter (ASU).

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5679393 A
- WO 2011122278 A
- EP 1246633 A
- FR 2678632
- FR 2762512